# EUROPÄISCHE PATENTSCHRIFT

(11) **EP 1 103 044 B1**
(45) Veröffentlichungstag und Bekanntmachung des Hinweises auf die Patenterteilung: **10.04.2002**
(21) Anmeldenummer: 99939432.3
(22) Anmeldetag: 02.08.1999
(51) Int. Cl.: G10K 11/00

(54) **ANORDNUNG ZUR WÄRMEABLEITUNG, INSBESONDERE FÜR ULTRASCHALLWANDLER MIT HOHER LEISTUNG**
HEAT DISSIPATING DEVICE, ESPECIALLY FOR HIGH PERFORMANCE ULTRASONIC TRANSDUCERS
DISPOSITIF DISSIPATEUR DE CHALEUR, EN PARTICULIER POUR TRANSDUCTEURS ULTRASONORES A HAUTES PERFORMANCES

(30) Priorität: 04.08.1998 DE 19836229
(43) Veröffentlichungstag der Anmeldung: 30.05.2001
(73) Patentinhaber: Dr. Hielscher GmbH, 14513 Teltow (DE)
(72) Erfinder: HIELSCHER, Harald, D-14532 Stahnsdorf (DE)
(74) Vertreter: Hengelhaupt, Jürgen D., Dipl.-Ing.
(86) Internationale Anmeldenummer: EP9905535
(87) Internationale Veröffentlichungsnummer: WO0008630

(56) Entgegenhaltungen:
- EP-A- 0 553 804
- EP-A- 0 782 125
- US-A- 5 545 942

## Beschreibung

Die Erfindung betrifft eine Anordnung zur Wärmeableitung, insbesondere für Ultraschallwandler mit hoher Leistung für die industrielle Anwendung.

Es ist allgemein bekannt, Ultraschallwandler hoher Leistung zu kühlen, um die Wärme abzuleiten, welche durch die Umwandlung der elektrischen Energie in mechanische Energie durch innere Reibung in den Piezoelementen und durch elektrische Verluste entsteht.

Die bekannten Kühlsysteme bestehen in vielen Fällen nur aus einem den Ultraschallwandler umgebenden Gehäuse mit Öffnungen, durch die Wärme durch Konvektion abgeführt wird (Prospekt der Firma Bandelin electronic "SONOREX Ultraschall-Desintegratoren", Sonopuls HD 60). Diese Art der Kühlung ist für hohe Leistungen unzureichend. Bei einer zusätzlichen Kühlung durch einen Lüfter werden Staub und Feuchtigkeit in das Gehäuse transportiert und die Gefahr von elektrischen Kurzschlüssen durch Brückenbildungen erhöht sich.

Es ist auch die Abführung der Wärme über eine am Schallwandler montierte Horn-Flansch-Gehäuse-Verbindung bekannt, wobei die Wärmeableitung über eine Kupfer-Kühlplatte für Wasserkühlung erfolgt (Prospekt der Firma TELSONIC "Ultraschall-Hochleistungs-Reaktor, Serie SRR"). Auch hier ist die Wärmeableitung für hohe Leistung und Dauerbetrieb unzureichend. Neben der schlechten Wärmeleitung von Titan kann bei dieser Anordnung auch nur eine schmale Verbindung zum Horn an der Stelle eines Schwingungsknotens (Nullstelle) realisiert werden, um einen schwingungsentkoppelten Übergang zu schaffen. Damit erfolgt ein nur geringer Wärmeübertrag von der Wärmequelle zum Kühlsystem, der für den Dauerbetrieb mit hohen Leistungen nicht ausreicht. Eine Übertragung von Schwingungen auf das Kühlsystem ist zu vermeiden, da Leistung verloren gehen und eine weitere Erwärmung erfolgen würden.

Es sind des weiteren zahlreiche Varianten der Kühlung bekannt, so zum Beispiel die Gehäuse mit Lüfterkühlung oder mit Preßluft zu versehen. Diese Systeme weisen ebenfalls die Gefahr des elektrischen Kurzschlusses auf. Auch geschlossene Systeme mit Lüfter und Wärmeaustausch von innen nach außen sind bekannt, die jedoch gerätetechnisch aufwendig sind und nur eine begrenzte Wärmeabfuhr ermöglichen.

In der DE 43 39 786 A1 wird eine Anordnung zur Wärmeableitung beschrieben, bei der zur Wärmeableitung von elektronischen Bauteilen unmittelbar auf der Oberfläche des Gehäuses des elektronischen Bauteiles ein wärmeleitender Kunststofformkörper aus einem Silikonpolymer vorgesehen ist, der das auch zur Kühlung dienende Gehäuse flächig kontaktiert.

In der DE 35 28 291 A1 wird eine Anordnung zur Kühlung elektronischer Bauelemente angegeben, bei der ein Schüttgut die Wärmeübertragung von den Bauteilen zu den Kühlkörpern übernimmt. Als Schüttgut sind Sand oder Glasperlen einer bestimmten Körnung vorgesehen.

Diese Anordnungen erfordern weiterhin ein aufwendiges Kühlsystem mit Kühlkörpern und den damit verbundenen Nachteilen.

Nachteilig ist es bei allen bekannten Lösungen, daß der Dauerbetrieb von Ultraschallwandlern bei hohen Leistungen, insbesondere auch in explosionsgeschützter Ausführung oder für Ausführungen in feuchter Umgebung, nicht ohne großen Aufwand und/oder ohne eine Verschlechterung des Wirkungsgrades gewährleistet werden kann.

Aufgabe der Erfindung ist es, eine Anordnung zur Wärmeableitung, insbesondere für Ultraschallwandler mit hoher Leistung, zu entwickeln, welche den Dauerbetrieb des Ultraschallwandlers mit hoher Leistung auch in Umgebungen mit hoher Feuchte und/oder Wärme und in explosionsgeschützter Ausführung durch eine effektivere Wärmeabführung als bisher bekannt zuverlässig gewährleistet.

Die Lösung dieser Aufgabe ergibt sich aus den Merkmalen des Anspruchs 1. Durch das den Ultraschallwandler umgebende geschlossene Kühlungssystem bestehend aus einer dünnen schwingungsabsorbierenden elastischen Schicht wie Silikonkautschuk und einer wärmeableitenden Schicht wie Quarzsand wird erreicht, daß keine mechanischen Verluste bei der Übertragung der Wärmeleistung auftreten, da die schwingungsabsorbierende Schicht keine Schwingungen auf die folgenden Schichten überträgt. Der direkte Auftrag der Schichten auf den Ultraschallwandler bewirkt eine effektive großflächige Wärmeableitung ohne großen und störanfälligen Geräteaufwand.

Zweckmäßige Ausgestaltungen der Erfindung sind in den Unteransprüchen angegeben.

Die Erfindung wird nachfolgend in einem Ausführungsbeispiel einer Anordnung zur Wärmeableitung für einen Ultraschallwandler anhand einer Zeichnung näher erläutert. Es zeigen:
- Fig. 1 :: eine schematische Schnittdarstellung des Ultraschallwandlers mit Kühlungssystem und
- Fig. 2 :: den Schnitt A-A durch den Ultraschallwandler nach Fig. 1.

Für Hochleistungs-Ultraschallwandler im kW-Bereich ist die Wärmeableitung von großer Bedeutung, um den zuverlässigen Dauerbetrieb in industriellen Anlagen zu gewährleisten.

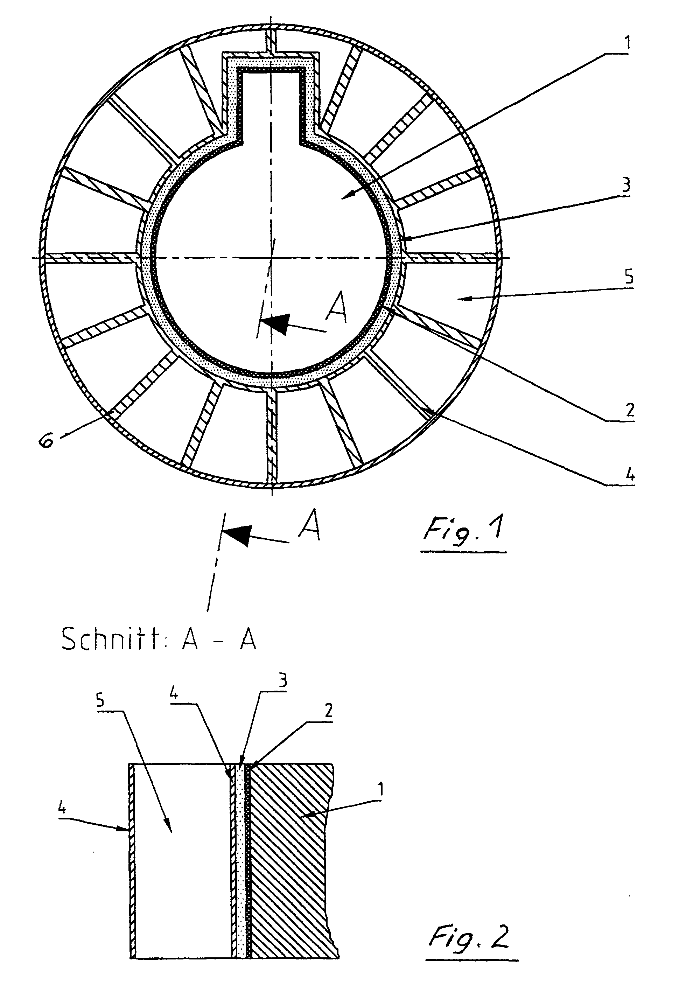

Aus der Schnittdarstellung der Figur 1 und der Detaildarstellung der Fig. 2 ist der Aufbau des Kühlungssystems für einen Ultraschallwandler 1 erkennbar.

Die Oberfläche des Ultraschallwandlers 1 ist mit einer elastischen schwingungsabsorbierenden Schicht 2, zum Beispiel aus Silikon-Kautschuk, in einer Schichtdicke von beispielsweise 0,05 mm bis 0,5 mm beschichtet.

Diese Schicht 2 nimmt die vom Ultraschallwandler abgestrahlten Schwingungen auf und absorbiert sie, so daß auf der Oberfläche der Schicht 2 keine Schwingungen auftreten.

Auf die Schicht 2 ist eine wärmeleitende Schicht 3 aufgebracht, die beispielsweise aus Quarzsand bestehen kann und beispielsweise eine Stärke von 0,2 mm bis 2 mm aufweist. Die Erwärmung des Ultraschallwandlers 1 wird über dessen gesamte Oberfläche auf die Schicht 2 und von dort auf die Schicht 3 abgeführt.

Die wärmeleitende Schicht 3 ist wiederum in engem Kontakt mit einem Gehäuse 4, beispielsweise aus Alu-Strangguß, verbunden, welches Kühlrippen und eine äußere Ummantelung aufweisen kann. Zwischen den Kühlrippen können wärmeleitende Medien wie Luft, Wasser oder Öl und dergleichen eingebracht sein. Weiterhin ist es möglich, von der Schicht 3 in bekannter Weise die Wärme durch Ventilation oder mittels anderer bekannter Kühlsysteme abzuziehen.

Das Kühlsystem erlaubt durch das geschlossene Gehäuse 4 eine explosionsgeschützte Ausführung des Ultraschallwandlers und/oder seine Anwendung im Dauerbetrieb in feuchter oder aggressiver Umgebung.

Die Erfindung ist nicht auf die hier beschriebenen Ausführungsformen beschränkt. Vielmehr ist es möglich, durch Kombination der Merkmale weitere Ausführungsbeispiele zu realisieren, ohne den Rahmen der Erfindung zu verlassen.

### Bezugszeichenliste

- 1: Ultraschallwandler
- 2: schwingungsabsorbierende Schicht
- 3: wärmeableitende Schicht
- 4: Gehäuse
- 5: Segment
- 6: Kühlrippe

## Patentansprüche

1. Anordnung zur Wärmeableitung, insbesondere für Ultraschallwandler mit hoher Leistung für die industrielle Anwendung,
**dadurch gekennzeichnet, daß**
eine schwingungsabsorbierende elastische Schicht (2) wie Silikonkautschuk und eine mit dieser Schicht (2) in direktem Kontakt stehende wärmeableitende Schicht wie Quarzsand ein Kühlungssystem bildet, welches das zu kühlende Objekt (1) umgibt.

2. Anordnung nach Anspruch 1,
**dadurch gekennzeichnet, daß**
das Kühlungssystem einen Ultraschallwandler (1) umschließt.

3. Anordnung nach den Ansprüchen 1 und 2,
**dadurch gekennzeichnet, daß**
die schwingungsabsorbierende Schicht (2) und auf diese Schicht (2) die wärmeableitende Schicht (3) in direktem Kontakt mit der Oberfläche des Ultraschallwandlers (1) aufgebracht sind, und daß die wärmeableitende Schicht (3) in engem Kontakt mit einem Gehäuse (4) zur großflächigen Ableitung der Wärme nach außen verbunden ist.

4. Anordnung nach Anspruch 3,
**dadurch gekennzeichnet, daß**
das Gehäuse (4) aus wärmeleitfähigem Material wie Aluminium besteht und Kühlrippen (6) mit dazwischenliegenden Segmenten (5) aufweist, durch die wärmetransportierende Medien wie Luft, Wasser, Öl geführt sind.

## Claims

1. Arrangement for heat discharge, particularly for ultrasonic transducers with high performance rating for the industrial application,
wherein,
a vibration-absorbing elastic layer (2) such as silicone caoutschouc and a heat discharging layer such as quartz sand, which is in direct contact with this layer (2), form a cooling system which surrounds the object (1) to be cooled.

2. Arrangement according to Claim 1,
wherein,
the cooling system encloses an ultrasonic transducer (1).

3. Arrangement according to the Claims 1 and 2,
wherein
the vibration-absorbing layer (2) and, onto this layer (2), the heat-discharging layer (3) in direct contact with the surface of the ultrasonic transducer (1) is applied, and the heat-discharging layer (3) is joined in close contact with a casing (4) for wide-surface discharge of the heat to the outside.

4. Arrangement according to Claim 3,
wherein,
the casing (4) consists of a heat-conductive material such as aluminium and has cooling fins (6) with segments (5) lying in between, through which heat-transporting media such as air, water, oil are conducted.

## Revendications

1. Dispositif de dissipation de chaleur, notamment pour convertisseur à ultrasons de haute puissance pour un usage industriel,
**caractérisé en ce qu'** une couche élastique (2) absorbant les oscillations telle que caoutchouc au silicone et une couche dissipatrice de chaleur en contact direct avec ladite couche (2), telle que sable siliceux, forment un système de refroidissement enveloppant l'objet (1) à refroidir.

2. Dispositif selon la revendication 1,
**caractérisé en ce que** le système de refroidissement enveloppe un convertisseur à ultrasons (1).

3. Dispositif selon les revendications 1 et 2,
**caractérisé en ce que** la couche absorbant les oscillations (2) et, sur celle-ci (2), la couche dissipatrice de chaleur (3) sont en contact direct avec la surface du convertisseur à ultrasons (1), et **en ce que** la couche dissipatrice de chaleur (3) est étroitement reliée à un boîtier (4) pour la dissipation superficielle de chaleur vers l'extérieur.

4. Dispositif selon les revendications 1 et 2,
**caractérisé en ce que** le boîtier (4) se compose d'une matière conductrice thermique telle que l'aluminium et présente des ailettes de refroidissement (6) avec segments intercalaires (5), par lesquelles sont conduits les milieux transporteurs thermiques comme l'air, l'eau et l'huile.
